# EUROPEAN PATENT SPECIFICATION

(11) **EP 3 676 309 B1**
(45) Date of publication and mention of the grant of the patent: **12.01.2022**
(21) Application number: 18765078.3
(22) Date of filing: 31.08.2018
(51) Int. Cl.: A61K 9/20, C08L 67/04, C08J 3/14, B33Y 70/00, B01F 3/08, B01F 5/06

(54) **BIOCOMPATIBLE POLYMER POWDERS FOR ADDITIVE MANUFACTURING**
BIOKOMPATIBLE POLYMERPULVER FÜR DIE ADDITIVE HERSTELLUNG
POUDRES POLYMÈRES BIOCOMPATIBLES POUR LA FABRICATION D'ADDITIFS

(30) Priority: 03.09.2017 US 201762553883 P; 11.09.2017 US 201762556944 P
(43) Date of publication of application: 08.07.2020
(73) Proprietor: Evonik Operations GmbH, 45128 Essen (DE)
(72) Inventor: GENTSCH, Rafael, 48163 Münster (DE); NIKOUKAR, Mohammad, Sunnyvale, California 94085 (US); ACREMAN, Kevin, Birmingham Alabama 35226 (US); PATEL, Harsh, 41091 Union, KY (US); SHAH, Milin, 30024 Suwanee, GA (US); TICE, Tom, 35124 Indian Springs, AL (US); KARAU, Andreas, 63571 Gelnhausen (DE); LIZIO, Rosario, 64839 Münster (DE); ZHANG, Jian-Feng, 35243 Vestavia, AL (US)
(74) Representative: Evonik Patent Association
(86) International application number: PCT/EP2018/073424
(87) International publication number: WO 2019/043137

(56) References cited:
- CN-A- 104 441 668
- US-A- 5 945 126
- US-A1- 2006 018 942
- US-A1- 2010 069 602
- US-A1- 2011 204 533
- US-A1- 2014 004 348
- US-B2- 8 916 196

## Description

### Field of the Invention

This invention relates to the manufacturing of biodegradable polymer powders for the use in additive manufactured objects like medical implants or scaffolds. The powders have biocompatible polymers and biodegradable osteoconductive flowability aids suited for medical applications. The composition of those powders enables easy processing at normal conditions of additive manufacturing like selective laser sintering to ensure decent mechanical part performance. The invention will allow to 3D print polymeric implantable medical devices with flexible and complex design meaning for example patient specific and porous geometries.

### Background of the Invention

Currently, most of the implantable medical devices are still produced using conventional manufacturing processes in particular the polymer space. Depending on the application of the devices, they will be molded (e.g. injection molding, compression molding), extruded, foamed, or will have multiple steps, such as extrusion combined with machining or laser cutting. However, there is a new trend to 3D print in the medical field to design highly controlled structures which can be customized to CT-Scan of patients e.g. after a trauma or loss of bone after cancer removal (D. J. Bonda et al, Neurosurgery 77:814-824, 2015).

3D printing or sometimes referred to as additive manufacturing or rapid manufacturing is a manufacturing class having in common a layer-by-layer build of a 3D objects using computer aided design software. Different material forms can be used such as powders, liquids and filaments, which correlate to the different additive manufacturing techniques as described for medical device in US005518680A. The focus of this invention is on powder based 3D printing for example selective laser sintering (SLS) or melting. The advantage of such systems is that no support structures are needed and multiple products with different geometries can be produced. Typically, the powder bed is heated close to the melting point. Mostly polyamide 12 (PA12) is used for SLS, which is used for different applications also in the medical field, but not for implantable devices. In this system, typically flowability aids are silicon oxides. They are being added to ensure proper flow at the processing conditions. However, the silicon oxide nanoscopic structure and chemistry is not suitable for implantable setups due to its toxicological concern. Therefore, different groups have looked into a pure polymer system, or a system that adds calcium phosphate, which are known to be biocompatible especially in the orthopedics field.

In the biocompatible polymer space different groups have started working on powder production for SLS for synthetic resorbables powders like polylactid acid (PLA, melting point of ~180°C) and polycaprolactone (PCL, melting point of ~60°C). However, these groups mostly use the milling particle route. In CN104441668A for example PCL was cryogenically milled down to 110 +/- 20 µm. This PCL was then mixed with 10-30% to Hydroxyapatite particles with 40+/-10nm in size for 1h. Then it was processed at 49°C bed temperature. Wiria et al (Acta Biomaterialia 3 (2007) 1-12) uses ground Capa6501 mixed with HA in the same concentration, but using highly crystalline HA with 5µm in size. The bed temperature used was 40°C. In addition, pure nonmedical grade PCL such as Capa6501 powder was used with 99% powder below 100 µm was processed at 46° and 48°C (S. J. Hollister et al, J Manufacturing Sci & Eng, 2006, 128, 531). In addition Shishkovsky et al (Physics Procedia 39 (2012) 491 - 499) explored also PCL, Polycarbonate (PC) and polyetheretherketon, but uses high doping of ceramic filler and resulted in coloration of the polymer upon selective laser sintering. Similarly Partec et al (Mater. Res. Soc. Symp. Proc. Vol. 845, 2005, Materials Research Society) also used high loading of TCP additive with particle size of 45 µm in PCL. Some groups also used ground PLLA like Tan et al (Bio-Medical Materials and Engineering 15 (2005) 113-124) adding HA powders with 90wt% below 60µm. The powder bed-temperature was held at 60°C.

Regarding multicomponent system CN103709737 also proposes different additives using nanoscaled flow aids, semiconductor materials like erbium-doped yttrium or organic antioxidant.

Besides milling, the emulsion route to produce microparticles was pursued. Du et al (Colloids and Surfaces B: Biointerfaces 135 (2015) 81-89) used a 20% HA concentrated PCL powder, where the inorganic is incorporated and therefore does not act as flow aid. In CN101138651, PLLA microsphere were also synthesized using emulsion and blending with 30-100µm NaCl powders (30-100µm) in a ratio of 2:1. Also 10% TCP was incorporate in the microparticles in another example in the same patent.

Other round-sphere shaped route are using a dispersion in the melt phase like described in JP4846425, where the matrix is being removed. Also in this case nanoscaled sized aids were used to improve flowability of such systems.

Overall, groups have used powders, which were processed at temperatures much lower than the melting point, which limits the part performance because the thermal stresses will build-up. In addition, some construct showed very porous structure, where the powder did not completely fuse, which will not be suited for mechanical challenging applications. This might also has to do with the rather unusual layer thickness of up to 0.3 µm, which some groups used. Some of the groups also use nanoscopic additives. However, the medical community has its concerns regarding the toxicological effects such additives have when handling the powders. (Yu et al, J Nanopart Res (2009) 11:15-24). Same holds true for materials systems with nonbiocompatible laser absorbant, antioxidant, softener or flow aids. In addition, flowability data are mostly generated at room temperature, which do not represent the flow characteristics close to its melt point. Furthermore, some of the parts did have a good surface finish, due to the poor flowability mostly using roll instead of blade suspension system, which are more tolerant when it comes to poor flowability.

There is still a need for a biocompatible powder, which can be processed under normal conditions similar to PA12 for industrial applications especially in the bioresorbable field.

### Summary of the Invention

It is an object of the present invention to provide biocompatible polymeric powders, which have good flow properties. In addition, these powders have good three dimensional printing (3D printing) characteristics with respect to processing and its resulting parts. Those powders are composed of cryogenically ground biocompatible polymer powders and of biocompatible flow aids.

3D printing methods could be powder based techniques like selective laser sintering (SLS), but also other methods like binder jetting, selective absorbing sintering (SAS) or hybrid methods of those.

With such printing methods different kind of medical implants could be produced with any inner and outer structure as well as decent mechanical properties compared to conventional manufacturing (e.g. injection molding) due to the proper sintering of particles together.

The advantage of the one aspect of the disclosure is that surprisingly those spherical powders with a narrow size distribution could be processed under normal conditions without any need of flow aids. Normally groups reduce temperatures to allow proper flow further away from the melting point. This either increases the thermal gradient in the powder bed which can lead to curling behavior or tension in the final part in particular for thicker objects. Both reduces the quality of the end part. The other strategy is the in particular R&D groups make sacrifices in the part homogeneity due to insufficient flow and limited to the roll based dispensing system as with blade based system such attempt will lead to build failure. This obviously is also a major drawback or current systems, which limits its application for parts in the body, which have to carry a certain load.

An aspect of the invention addresses the nature of the flow aids, which are used in irregular shaped particles as well as in some round-shaped powders. As mention in the background section different data has shown using additive, such as HA and TCP, as being biocompatible calcium phosphates. In addition, groups have used typically sizes similar to the polymer counterpart also at high loadings of 10 or more wt% or nanosized versions, which tend to clump together, or be a concern from a toxicological point of view. Therefore, flow aids were selected having a submicron size with a decent particle size distribution to alleviate those concern. Furthermore, it was shown astonishingly that such additives using unsintered or partially amorphous state will significantly outperform the commonly used crystalline versions in terms of flow properties through a comparative study. This holds true in particular closer to the printing conditions (i.e. temperature close to the melt point of the polymer). This is another reason why groups have used lower bed temperatures such as described in the background section in particular for polylactide powders. Using temperatures considerably lower than the melting point will have shortcomings in processing or part performance as described above.

### Brief Description of the Drawings

The above and other objects, features and advantages of the present invention will be more clearly understood from the following detailed description taken in conjunction with the accompanying drawings, in which:
FIG. 1- Flow results of PCL powder blends with unsintered additives.
FIG. 2- Flow results of PDO powder blends with unsintered additives.
FIG. 3- Flow results of PLA powder blends with unsintered additives.
FIG. 4- Effects of unsintered and sintered additive use on PDO powder flow.
FIG. 5- Effects of unsintered and sintered additive use on PCL powder flow.

### Detailed Description of the Invention

Before the present powders and processes are disclosed and described, it is to be understood that the aspects described herein are not limited to specific processes, compounds, synthetic methods, articles, devices, or uses as such can, of course, vary. It is also to be understood that the terminology used herein is for the purpose of describing particular aspects only and, unless specifically defined herein, is not intended to be limiting.

Disclosed herein are biocompatible polymers powders that show improved powder performance such as flowability and 3D printing characteristics. Those will enable an easy additive manufacturing of medical implants in many field of orthopedics (such as e.g. craniomaxillofacial, extremities, trauma, spine, orthobiologics), cardiovascular, dental, plastic surgery, pulmonology, thoracic surgery, regenerative medicine etc.

### Definition of Terms

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention.

The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "an" and "the" include plural references unless the context clearly dictates otherwise. The present disclosure also contemplates other embodiments "comprising," "consisting of" and "consisting essentially of," the embodiments or elements presented herein, whether explicitly set forth or not.

The conjunctive term "or" includes any and all combinations of one or more listed elements associated by the conjunctive term. For example, the phrase "an apparatus comprising A or B" may refer to an apparatus including A where B is not present, an apparatus including B where A is not present, or an apparatus where both A and B are present. The phrases "at least one of A, B, . . . and N" or "at least one of A, B, ... N, or combinations thereof" are defined in the broadest sense to mean one or more elements selected from the group comprising A, B, ... and N, that is to say, any combination of one or more of the elements A, B, ... or N including any one element alone or in combination with one or more of the other elements which may also include, in combination, additional elements not listed.

The modifier "about" used in connection with a quantity is inclusive of the stated value and has the meaning dictated by the context (for example, it includes at least the degree of error associated with the measurement of the particular quantity). The modifier "about" should also be considered as disclosing the range defined by the absolute values of the two endpoints. For example, the expression "from about 2 to about 4" also discloses the range "from 2 to 4." The term "about" may refer to plus or minus 10% of the indicated number. For example, "about 10%" may indicate a range of 9% to 11%, and "about 1" may mean from 0.9-1.1. Other meanings of "about" may be apparent from the context, such as rounding off, so, for example "about 1" may also mean from 0.5 to 1.4.

The term "wt. %" means weight percent.

The terms "microencapsulated" and "encapsulated" are used herein to refer generally to a bioactive agent that is incorporated into any sort of long-acting formulation or technology regardless of shape or design; therefore, a "microencapsulated" or "encapsulated" bioactive agent may include bioactive agents that are incorporated into a particle or a microparticle and the like or it may include a bioactive agent that is incorporated into a solid implant and so on.

The term "bioactive agent" is used herein to include a compound of interest contained in or on a pharmaceutical formulation or dosage form that is used for pharmaceutical or medicinal purposes to provide some form of therapeutic effect or elicit some type of biologic response or activity. "Bioactive agent" includes a single such agent and is also intended to include a plurality of bioactive agents including, for example, combinations of two or more bioactive agents.

The term "biocompatible" as used herein refers to a material that is generally non-toxic to the recipient and does not possess any significant untoward effects to the subject and, further, that any metabolites or degradation products of the material are non-toxic to the subject. Typically a substance that is "biocompatible" causes no clinically relevant tissue irritation, injury, toxic reaction, or immunological reaction to living tissue.

The term "biodegradable" as used herein refers to a material that will erode to soluble species or that will degrade under physiologic conditions to smaller units or chemical species that are, themselves, non-toxic (biocompatible) to the subject and capable of being metabolized, eliminated, or excreted by the subject.

The term "non-agglomerating" as used herein refers to the powder when it does not generate large clumps of material that are created by the presence of moisture or packing of the powder.

The term "improved flowability" as used herein refers to the fluidization of powder particulates through movement generated by various stimuli to include impact forces from falling, dynamic powder flow analysis, and especially spreading by blade, roller, or feeding mechanism during processing. An improved flowability is reference to a decrease in break energy or avalanche energy after a base powder is treated with the flow aid.

The term "powder processing technologies" as used herein refers to but are not limited to, technologies such as SLS 3D printing, powder fluidization, powder compounding, powder feeding, etc.

The term "avalanche energy" as used herein refers to the power change of a powder during an avalanche.

The term "break energy" as used herein refers to the energy at which the powder avalanches.

The term "interparticulate friction" as used herein refers to the friction force present between two contacting polymer particle surfaces. The additive reduces the surface area in contact, which will reduce the amount of friction between particles and will result in a more fluidized or more flowable powder.

The term "hausner ratio" as used herein refers to the ratio between the bulk density and the tap density of the powder.

The term "compressibility index" as used herein refers to the a percentage representation of how much the powder compresses during the tap density measurement.

Polymer powders could be any biocompatible polymers. Biocompatible polymers include but are not limited to polyarylketones, polymethacrylates, polycarbonates, polyacetals, polyethylenes, polypropylenes, polylactides, polydioxanones, polycaprolactones, polyesteramide, polyurethane, polytrimethyl carbonates, polyglycolide, poly(amio acid) and copolymers of the respective monomers like poly(lactide-co-glycolide), poly(lactide-co-caprolactone), poly(lactide-co-trimethly carbonate), poly(lactide-co-polyethylene-glycol), poly(orthoester), a poly(phosphazene), a poly(hydroxybutyrate) a copolymer containing a poly(hydroxybutarate), a poly(lactide-co-caprolactone), a polyanhydride, a poly(dioxanone), a poly(alkylene alkylate), a copolymer of polyethylene glycol and a polyorthoester, a biodegradable polyurethane, a polyamide, a polyetherester, a polyacetal, a polycyanoacrylate, a poly(oxyethylene)/poly(oxypropylene) copolymer, polyketals, polyphosphoesters, polyhydroxyvalerates or a copolymer containing a polyhydroxyvalerate, polyalkylene oxalates, polyalkylene succinates, poly(maleic acid), and copolymers, terpolymers, combinations thereof.

Lactide-based polymers can comprise any lactide residue, including all racemic and stereospecific forms of lactide, including, but not limited to, L-lactide, D-lactide, and D,L-lactide, or a mixture thereof. Useful polymers comprising lactide include, but are not limited to poly(L-lactide), poly(D-lactide), and poly(DL-lactide); and poly(lactide-co-glycolide), including poly(L-lactide-co-glycolide), poly(D-lactide-co-glycolide), and poly(DL-lactide-co-glycolide); or copolymers, terpolymers, combinations, or blends thereof. Lactide/glycolide polymers can be conveniently made by melt polymerization through ring opening of lactide and glycolide monomers. Additionally, racemic DL-lactide, L-lactide, and D-lactide polymers are commercially available. The L-polymers are more crystalline and resorb slower than DL polymers. In addition to copolymers comprising glycolide and DL-lactide or L-lactide, copolymers of L-lactide and DL-lactide are commercially available. Homopolymers of lactide or glycolide are also commercially available.

In a particular aspect, when the biodegradable polymer is poly(lactide-co-glycolide) or any other copolymers (mentioned above), or a mixture of poly(lactide) and poly(glycolide), the amount of lactide and glycolide in the polymer can vary. In a further aspect, the biodegradable polymer contains 0 to 100 mole %, 40 to 100 mole %, 50 to 100 mole %, 60 to 100 mole %, 70 to 100 mole %, or 80 to 100 mole % lactide and from 0 to 100 mole %, 0 to 60 mole %, 10 to 40 mole %, 20 to 40 mole %, or 30 to 40 mole % glycolide, Wherein the amount of lactide and glycolide is 100 mole %. In a further aspect, the biodegradable polymer can be poly(lactide), 95:5 poly(lactide-coglycolide) 85:15 poly(lactide-co glycolide), 75:25 poly(lactide-co-glycolide), 65:35 poly(lac tide-coglycolide), or 50:50 poly(lactide-co-glycolide), Where the ratios are mole ratios.

In another aspect, the polymer can be a poly(caprolactone) or a poly(lactide-co-caprolactone). In one aspect, the polymer can be a poly(lactide-caprolactone), Which, in various aspects, can be 95:5 poly(lactide-co-caprolactone), 85:15 poly(lactide-co-caprolactone), 75:25 poly(lactide-co-caprolactone), 65:35 poly(lactide-co-caprolactone), or 50:50 poly (lactide-co-caprolactone), where the ratios are mole ratios.

In one aspect of the disclosure, a solvent process is being used to yield round-shaped powders which is described in US2010/0069602A1 and US2007/0207211A1. Briefly a dispersant phase having the dissolved polymer is being passed through with a continuous phase having no solubility with the polymer a packed bed apparatus under laminar flow conditions for powder production using emulsion based technique. At least one salt is being added to the process, preferably to the continuous phase reducing the solubility of the first solvent in the second solvent due improve the flowability of the final powder. This salt is not a flow aid, but rather a processing aid during the emulsion process.

In addition, filler can be added to the process to embed or encapsulate functionalities in the polymer matrix such as bioactive molecules like a drug, biomolecules or bioceramics as shown in another embodiment. Those fillers are physically bound to the polymer material and are not free-flowing.

Flow aids and bioceramics include but are not limited to calcium phosphate and its doped variants (e.g. strontium, zinc, magnesium, fluoride, carbonate), calcium sulfate, calcium carbonate, biocompatible glasses such as bioglass etc. Calcium phosphate include but are not limited to hydroxyapatite, tricalcium phosphate, calcim deficient carbonate-containing hydroxyapatite, octacalcium phosphate, dicalcium phosphate, biphasic calcium phosphate or a mixture thereof.

In one aspect of the invention, cryogenically ground polymer powders are being used as base powder. In order to improve their flow behavior flow aids had to be added, such as bioceramics. Interestingly, particularly effective flow aids seem to have in common a low-crystalline (unsintered) form of bioceramics. This feature enables a rather homogeneous mixing behavior and good flow properties. The mixing with the polymer powder can be done on a tumbler mixer or a high shear mixer. These mixers are commercially available from Somakon and other companies.

Such flow aids are in the range of 0.1-10µm size. Typical concentrations of such polymer based powder are range between 0.1-10wt% flow aids. Also mixtures of different flow aids can be used.

Such flow aids can be used for different kind of particle shapes. Such shapes are but are not limited to irregular shaped polymer powder, and spherical shaped polymer powder.

A wide variety of bioactive agents can be used with the methods described herein. In one aspect, the bioactive agent can be a releasable bioactive agent, i.e., a bioactive agent that can be released from the controlled release system into adjacent tissues or fluids of a subject. In certain aspects, the bioactive agent can be in or on the controlled release system.

Various forms of the bioactive agent can be used, which are capable of being released from the controlled release system into adjacent tissues or fluids. To that end, a liquid or solid bioactive agent can be incorporated into the controlled release system described herein. The bioactive agents are at least very slightly water soluble, and preferably moderately water soluble. The bioactive agents can include salts of the active ingredient. As such, the bioactive agents can be acidic, basic, or amphoteric salts. They can be nonionic molecules, polar molecules, or molecular complexes capable of hydrogen bonding. The bioactive agent can be included in the compositions in the form of, for example, an uncharged molecule, a molecular complex, a salt, an ether, an ester, an amide, polymer drug conjugate, or other form to provide the effective biological or physiological activity.

Examples of bioactive agents that incorporated into systems herein include, but are not limited to, peptides, proteins such as hormones, enzymes, antibodies and the like, nucleic acids such as aptamers, iRNA, DNA, RNA, antisense nucleic acid or the like, antisense nucleic acid analogs or the like, low-molecular weight compounds, or high-molecular-weight compounds. Bioactive agents contemplated for use in the disclosed implantable composites include anabolic agents, antacids, anti-asthmatic agents, anti-cholesterolemic and anti-lipid agents, anti-coagulants, anti-convulsants, anti-diarrheals, anti-emetics, anti-infective agents including antibacterial and antimicrobial agents, anti-inflammatory agents, anti-manic agents, antimetabolite agents, anti-nauseants, anti-neoplastic agents, anti-obesity agents, anti-pyretic and analgesic agents, anti-spasmodic agents, antithrombotic agents, anti-tussive agents, anti-uricemic agents, anti-anginal agents, antihistamines (e.g., terfenadine), appetite suppressants, biologicals, cerebral dilators, coronary dilators, bronchiodilators, cytotoxic agents, decongestants, diuretics, diagnostic agents, erythropoietic agents, expectorants, gastrointestinal sedatives, hyperglycemic agents, hypnotics, hypoglycemic agents, immunomodulating agents, ion exchange resins, laxatives, mineral supplements, mucolytic agents, neuromuscular drugs, peripheral vasodilators, psychotropics, sedatives, stimulants, thyroid and anti-thyroid agents, tissue growth agents, uterine relaxants, vitamins, or antigenic materials.

Other bioactive agents include androgen inhibitors, polysaccharides, growth factors (e.g., a vascular endothelial growth factor-VEGF), hormones, anti-angiogenesis factors, dextromethorphan, dextromethorphan hydrobromide, noscapine, carbetapentane citrate, chlophedianol hydrochloride, chlorpheniramine maleate, phenindamine tartrate, pyrilamine maleate, doxylamine succinate, phenyltoloxamine citrate, phenylephrine hydrochloride, phenylpropanolamine hydrochloride, pseudoephedrine hydrochloride, ephedrine, codeine phosphate, codeine sulfate morphine, mineral supplements, cholestryramine, N-acetylprocainamide, acetaminophen, aspirin, ibuprofen, phenyl propanolamine hydrochloride, caffeine, guaifenesin, aluminum hydroxide, magnesium hydroxide, peptides, polypeptides, proteins, amino acids, interferons, cytokines, and vaccines.

Representative drugs that can be used as bioactive agents include, but are not limited to, peptide drugs, protein drugs, desensitizing materials, antigens, anti-infective agents such as antibiotics, antimicrobial agents, antiviral, antibacterial, antiparasitic, antifungal substances and combination thereof, antiallergenics, androgenic steroids, decongestants, hypnotics, steroidal anti-inflammatory agents, anti-cholinergics, sympathomimetics, sedatives, miotics, psychic energizers, tranquilizers, vaccines, estrogens, progestational agents, humoral agents, prostaglandins, analgesics, antispasmodics, antimalarials, antihistamines, cardioactive agents, nonsteroidal anti-inflammatory agents, antiparkinsonian agents, antihypertensive agents, β-adrenergic blocking agents, nutritional agents, and the benzophenanthridine alkaloids. The agent can further be a substance capable of acting as a stimulant, sedative, hypnotic, analgesic, anticonvulsant, and the like.

Other bioactive agents include but are not limited to analgesics such as acetaminophen, acetylsalicylic acid, and the like; anesthetics such as lidocaine, xylocalne, and the like; anorexics such as dexadrine, phendimetrazine tartrate, and the like; antiarthritics such as methylprednisolone, ibuprofen, and the like; antiasthmatics such as terbutaline sulfate, theophylline, ephedrine, and the like; antibiotics such as sulfisoxazole, penicillin G, ampicillin, cephalosporins, amikacin, gentamicin, tetracyclines, chloramphenicol, erythromycin, clindamycin, isoniazid, rifampin, and the like; antifungals such as amphotericin B, nystatin, ketoconazole, and the like; antivirals such as acyclovir, amantadine, and the like; anticancer agents such as cyclophosphamide, methotrexate, etretinate, and the like; anticoagulants such as heparin, warfarin, and the like; anticonvulsants such as phenyloin sodium, diazepam, and the like; antidepressants such as isocarboxazid, amoxapine, and the like; antihistamines such as diphenhydramine HCI, chlorpheniramine maleate, and the like; hormones such as insulin, progestins, 17-alpha-hydroxyporgesterone caproate, iso-allo-pregnanolonetestosterone, prenisolone, prednisone, dexamethasone estrogens (e.g.,. estradiol), corticoids, glucocorticoids, androgens, and the like; tranquilizers such as thorazine, diazepam, chlorpromazine HCI, reserpine, chlordiazepoxide HCI, and the like; antispasmodics such as belladonna alkaloids, dicyclomine hydrochloride, and the like; vitamins and minerals such as essential amino acids, calcium, iron, potassium, zinc, vitamin B₁₂, and the like; cardiovascular agents such as prazosin HCI, nitroglycerin, propranolol HCI, hydralazine HCI, pancrelipase, succinic acid dehydrogenase, and the like; peptides and proteins such as LHRH, somatostatin, calcitonin, growth hormone, glucagon-like peptides, growth releasing factor, angiotensin, FSH, EGF, bone morphogenic protein (BMP), erythopoeitin (EPO), interferon, interleukin, collagen, fibrinogen, insulin, Factor VIII, Factor IX, ENBREL^{®}, RITUXAM^{®}, HERCEPTIN^{®}, alpha-glucosidase, Cerazyme/CEREDOSE^{®}, vasopressin, ACTH, human serum albumin, gamma globulin, structural proteins, blood product proteins, complex proteins, enzymes, antibodies, monoclonal antibodies, and the like; prostaglandins; nucleic acids; carbohydrates; fats; narcotics such as morphine, codeine, and the like, psychotherapeutics; anti-malarials, L-dopa, diuretics such as furosemide, spironolactone, and the like; antiulcer drugs such as rantidine HCI, cimetidine HCI, and the like, and calcium channel antagonist such as nimodipine and the like, lumefantrine, cilengitide, 3-hydroxy-3-methylglutaryl-coenzyme A reductase inhibitors such as lovastatin and the like.

Biomolecules include but are not limited to fibrin, fibrinogen, cellulose, starch, collagen and hyaluronic acid.

The characterization of the different powders were done on standard test machine like laser scattering machine for particle size determination (commercially available from the company Beckmann coulter or Malvern). The DSC were performed with a heating rate of 10°/min and a cooling rate of 20°/min using a TA instruments machine. SEM was performed on a desktop machine (commercially available from the company Hitachi). XRD measurements were supplied by Himed. BET measurements were done on micromeritics instrument.

Selective laser sintering (SLS) was performed on blade based (e.g. EOS P series) and roll-based machines, which show similar tendencies with different sensitivitiy with regarding flowability and processing. The technique produces physical parts through a selective solidification of a variety of fine powders. SLS builds up parts layer by layer using powders which are selectively bonded when a laser beam scans the powder across each layer's cross sectional area. Typical laser energy densities are anywhere from 7 J/m² to 60 J/m² SLS fuses thin layers of powder (typically -0.1 mm thick) which have been spread across the build area using a counter-rotating roller or a blade. The part building takes place inside an enclosed chamber filled with an inert gas, preferably nitrogen gas, to minimize oxidation and degradation of the powdered material. The powder in the build platform is maintained at an elevated temperature just below the melting point (for semicrystalline polymer only) of the powdered material.

The quantitative methods used for characterizing powder flowablity involved use of a flowablity testing apparatus (mercury instruments) consisting of a rotating cylindrical drum with glass walls which characterizes movement of the powder particles with a camera. The apparatus measures the maximum height of the powder pile before occurrence of an avalanche. The higher powder build up before the weight of the powder causes an avalanche, then the more energy it requires the make the powder particles to flow. This value is referred to as break energy and it is an indication of the minimum energy required to make the powder particles flow. If not otherwise mentioned the test are being performed at ambient conditions (rt, humidity level around 40%).

The determination of the particle size was performed according to the United States Pharmacopeia 36 (USP) chapter <429> and European Pharmacopeia 7.0 (EP) chapter 2.9.31. The particle size distribution was determined utilizing a laser scattering instrument (e.g. Fa. Sympatec GmbH, type HELOS equipped with RODOS dry dispersing unit). The laser diffraction method is based on the phenomenon that particles scatter light in all directions with an intensity pattern that is dependent on particle size. A representative sample, dispersed at an adequate concentration in a suitable liquid or gas, is passed through the beam of a monochromic light source usually from a laser. The light scattered by the particles at various angles is measured by a multi-element detector, and numerical values relating to the scattering pattern are then recorded for subsequent analysis. The numerical scattering values are then transformed, using an appropriate optical model and mathematical procedure, to yield the proportion of total volume to a discrete number of size classes forming a volumetric particle size distribution (e.g. d₅₀ describes a particle diameter corresponding to 50% of cumulative undersize distribution).

### Examples

The following examples are put forth so as to provide those of ordinary skill in the art with a complete disclosure and description of how the compounds, compositions, articles, devices and/or methods claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in ° C. or is at ambient temperature, and pressure is at or near atmospheric.

### REFERENCE EXAMPLE 1

Spherical polylactic acid (PLLA, RESOMER^{®}L206S commercially available from Evonik) microparticles were produced using a solvent based emulsion process. A first phase containing 10 % w/w polylactic acid was prepared by dissolving 20 g polylactic acid in 180 g of dichloromethane. A second phase was prepared by dissolving 6 g of poly(vinyl alcohol) (PVA) in 10.8 g of dichloromethane and 594 g of water. The second phase was pumped through a packed bed apparatus (6mm stainless steel tubing, 150 mm long filled with 650 µm glass beads) at a rate of 20 mL/min. The first phase was pumped at the same time through the same packed bed apparatus at a flow rate of 5 mL/min. The emulsion was collected in excess volume of water and the particles were allowed to harden by removing the solvent. The column was packed, the phases were pumped, the emulsion was collected, and the solvent was removed to form microparticles as described in U.S. Patent No. 8,916,196. The powders were produced both using a salt (NaCI) as a process aid as well as without using a salt (NaCI) for comparison control.

The second batch was prepared using the same method as the one described above. In addition, the continuous phase was saturated with 2M NaCl and used for the emulsion process. The method of saturating the continuous phase with salt was performed as described in U.S. Publication No. 2010/0069602. The yield of the microparticle production was increased from 82.5% to 92.1% with the use of a salt as a process aid.

To compare this emulsion process of producing microparticles to the conventional product (milled product), PLLA material was cryogenically milled. The bulk density of the emulsified PLLA powders was 0.6 g/mL.

Particle size of the powders was determined using laser scattering. The particle sizes are depicted in Table 1. The spherical PLLA microparticles produced with salt has a d50 of 76 µm and a d10 - d90 range of 55-97 µm while the spherical PLLA microparticles produced without salt has a d50 of 73 µm and a d10-d90 range of 51-109 µm.The milled PLLA powder has a d50 of 54 µm and a d10 - d90 range of 23-103 µm.

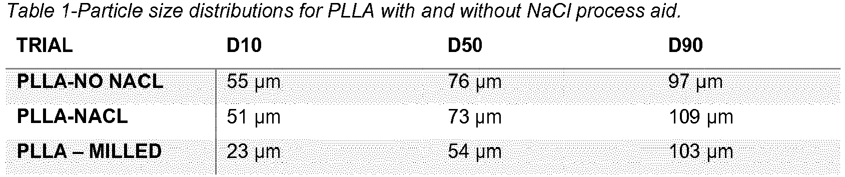

The thermal characteristics for the spherical PLLA emulsion microparticles and the milled PLLA microparticles were evaluated using differential scanning calorimetry (DSC). The DSC data does confirm that while the processes are different, the two different types of materials did not undergo a large thermal change during processing from granulate form. The largest difference seen is that the emulsion PLLA microparticles do experience a reduction in crystallinity compared to the granulate and milled form of the polymer. DSC data for spherical, milled, and granulate PLLA can be seen in Table 2.

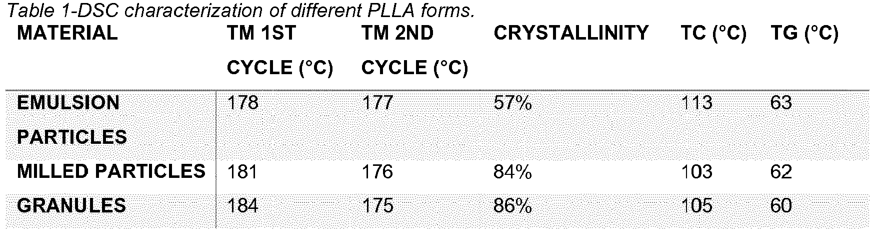

Initial flowability was assessed using a qualitative blade method within a commercially available SLS machine. A 'good' result from a blade test is when the blade produces a smooth and flat surface of powder. This is critical to the printing process in order to produce a dense and dimensionally accurate print. Initial flowability characteristics were poor for spherical PLLA microparticles produced without the use of salt. Spherical PLLA microparticles produced using salt as a process aid indicated good flow from the blade test without the need of a flow additive or additional treatment. The blade test produced a flat surface that could be utilized for the SLS printing application.

The spherical PLLA microparticles produced with salt indicated superior flowability. Milled PLLA polymer microparticles were used as a control. The milled powder did have a good blade result. Some avalanching was observed. This is caused by excess material falling onto an uneven surface. Avalanching can be defined as a shift in the powder surface. The smooth surface of the powder is the main indication of flow during the printing process, as layers will be close to 100 µm. After production of the spherical PLLA powders, the flow was evaluated using quantitative flow analysis. Avalanche energy and break energy were measured as depicted in Table 3. Avalanche energy represents the power change of a powder during an avalanche while the break energy represents the energy at which the powder avalanches. The spherical powder was compared to a milled control produced as described above from the same base resin. The avalanche energy for milled powder is 40 kJ/kg while the break energy for the milled powder is 131 kJ/kg. The avalanche energy for the spherical powder is 52 kJ/kg while the break energy for the milled powder is 75 kJ/kg. The break energy result represents that the spherical powder requires less energy to generate an avalanche and the larger avalanche energy indicates a larger shift in the powder surface. As a result, the spherical powder is shown to have better flow. The improvement in flow can also be referenced to as a reduction in interparticulate friction. Interparticulate friction is the friction force present between two contacting polymer particle surfaces. A larger surface area contact between particles results in a higher friction force to overcome in order to flow.

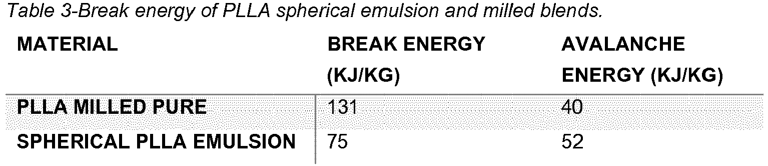

### REFERENCE EXAMPLE 2

Spherical Polycaprolactone (PCL, Evonik RESOMER^{®} C209 commercially available from Evonik) microparticles were produced by using the same solvent process including NaCl as in Example 1. A first phase containing 15 % w/w Polycaprolactone (PCL, Evonik RESOMER^{®} C209 commercially available from Evonik) by dissolving 25 g Polylactic acid Polycaprolactone (PCL, Evonik RESOMER C209) in 142 g of dichloromethane. The second phase was prepared by dissolving 6 g of poly(vinyl alcohol) (PVA) and 10.8 g of dichloromethane in 594 g of water. In addition, the continuous phase was saturated with 2M NaCl and used for the emulsion process. The second phase was pumped through a packed bed apparatus (6 mm stainless steel tubing, 150 mm long filled with 650 µm glass beads) of this invention at 20 mL/min. The first phase was pumped at the same time through the same packed bed apparatus at a flow rate of 5 mL/min. The emulsion was collected in excess volume of water and allowed the particles to harden by removing the solvent. The column was packed, the phases were pumped, the emulsion was collected, and the solvent was removed to form microparticles as described in U.S. Patent No. 8,916,196. The method of adding the salt to the continuous phase was performed as described in U.S. Publication No. 2010/0069602.

To compare this emulsion process of producing microparticles to the conventional product (milled product), PCL material was cryogenically milled. The particle size distribution of the emulsion and milled PCL powders was analyzed. The spherical emulsion PCL microparticles had a narrow range of 51 µm, while the milled PCL microparticles had a range of 69 µm. The milled PCL microparticles have a smaller d50 value of 49 µm compared to the spherical emulsion PCL microparticles with 81 µm. The PCL emulsion microparticles experienced a smaller particle size distribution than milled powders as shown in example 1 for PLLA. The particle size for emulsion can easily be adjusted to other particle size ranges using the packed bed apparatus process. All particle size data for the milled and spherical emulsion PCL microparticles can be seen in Table 4. The bulk density of the emulsified PCL powders was 0.54 g/mL.

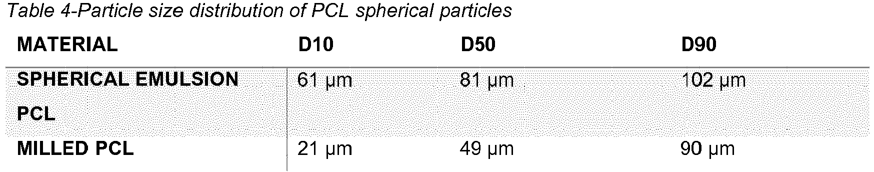

The thermal characteristics for the spherical PCL emulsion microparticles and the milled PCL microparticles were evaluated using differential scanning calorimetry (DSC). The milled and spherical emulsion PCL microparticles were similar in thermal profile. Both powders varied from the granulate form due to an increase in crystallization temperature (Tc) after processing as shown in Table 5.

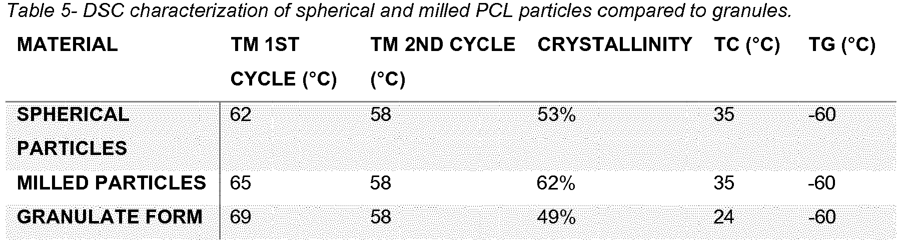

Initial flowability was assessed using a qualitative blade method within a commercially available SLS machine. A 'good' result from a blade test is when the blade produces a smooth and flat surface of powder. This is critical to the printing process in order to produce a dense and dimensionally accurate print. A 'poor' blade test result indicates that a single, flat, and uniform surface was not generated. In this case, the surface would indicate various pitting or show large amounts of agglomeration. The milled PCL microparticles had a poor blade test result. The surface generated had pitting and various observable agglomerates. The spherical emulsion PCL microparticles had a good blade test result.

The flowability of the spherical PCL microparticles and the milled PCL microparticles was evaluated by measuring break energy (kJ/kg). The spherical emulsion PCL microparticles showed a good blade test result while the milled material did not. This can also be seen through the break energy measurements. The spherical emulsion PCL microparticles had a break energy of 119 kJ/kg compared to the 126 kJ/kg result for the milled PCL microparticles. The avalanche energy for milled powder is 31 kJ/kg, and the avalanche energy for the spherical powder is 63.21 kJ/kg. This result, in combination with the blade test evaluation and a successful print operation, indicate that the spherical emulsion PCL microparticles are a superior printing powder compared to the pure milled form of the same polymer. The break energy quantitative result of flow can be seen in Table 6. This represents that the spherical powder requires less energy to generate an avalanche and the larger avalanche energy indicates a larger shift in the powder surface. As a result, the spherical powder is shown to have better flow.

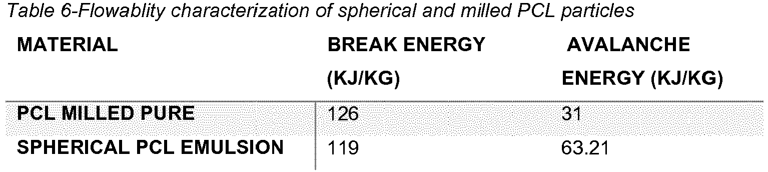

Good flowablity of the spherical PCL particles was also observed at the higher temperatures close to Tm of the polymer, which confirms the experiments at room temperature. In addition, easy processing of powders without any curling were observed. A commercially available SLS machine was used to print some tensile specimen out of the spherical PCL powder. A good surface finish and part accuracy was achieved which is important for medical applications.

### EXAMPLE 3

A polycaprolactone (PCL, RESOMER^{®} C209) milled powder was produced by cryogenically milling a granulate form with a mill. The milling protocol utilized a two cycle milling process of which the machine rotor reached speeds of up to 15000 rpm and the door rotor reached speeds of 11000 rpm. Through this process, 94% of the milled PCL material was able to be produced within the required specification range. The d50 particle size for the milled PCL is 55µm and the particle size distribution is shown Example 2.

A blend of unsintered hydroxyapatite (HA) additive manufactured by Hitemco Medical Application, Inc. (dba Himed, d50=0.8-0.9 µm) with milled Polycaprolactone (PCL) polymer powder was produced. A blend of unsintered beta-tricalcium phosphate (TCP) additive manufactured by Hitemco Medical Application, Inc. (dba Himed, d50=0.8-0.9 µm) with milled Polycaprolactone (PCL) polymer powder was produced. As a control, a blend of sintered hydroxyapatite (HA) from the same supplier (dba Himed, d50=0.5-0.75 µm) with milled PCL was produced. As a control, a blend of sintered TCP from the same supplier (dba Himed, d50=0.5-0.75 µm) with milled PCL was produced. All additives were measured with BET for surface area. Sintered HA (6.8m²/g) and sintered TCP (6.1m²/g) had a considerably smaller surface area than unsintered versions (HA: 57.5m²/g, TCP: 52.4m²/g). Multiple concentrations of sintered and unsintered HA were evaluated.. Blends were mixed through tumble mixing and high shear mixing machinery. By adding the additive to the milled PCL powder the bulk density increased compared to pure milled powder (0.25g/mL). Also the values of using unsintered TCP (0.47g/mL) and HA (0.50g/mL) were higher versus sintered TCP (0.40g/mL) and HA (0.37g/mL).

The thermal profile of the powders was analyzed using differential scanning calorimetry (DSC). The thermal profile of the material changed slightly from granulate to milled form through an increase in crystallinity and crystallization temperature (Tc). Also, a 4°C degree decrease in melt temperature (Tm) was observed. The powder blends that contained HA did see an increase in Tc, but the melt and crystallization temperatures did not indicate a significate change. The DSC data can be seen in Table 7. X-ray diffraction data from the HA additive indicates that the DSC may be giving a false value for crystallinity when powders are tested as a blend. X ray diffraction data can be seen in FIG. 7.

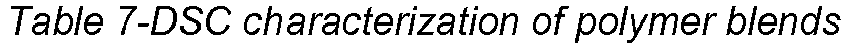

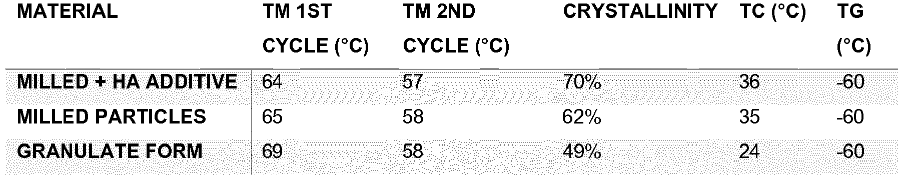

Qualitative testing of powder flow within a commercially available SLS machine was performed with all three powders. A 'good' result from a blade test is when the blade produces a smooth and flat surface of powder. This is critical to the printing process in order to produce a dense and dimensionally accurate print. Both the sintered HA and pure milled PCL mixtures had poor blade test results. Pitting and agglomerates were observed within the powder surface. Unsintered HA/milled PCL microparticle powder mixture had a good blade test result.

The quantitative characterization of the powder flowability was performed through measuring the break energy for pure particles, particles with 5 wt% of unsintered HA and particle with 5 wt % of sintered HA as depicted in Table 8. The milled PCL microparticles had a break energy of 126 kJ/kg. This break energy value was improved by adding unsintered HA but not by adding sintered HA. Unsintered HA reduced the break energy of the milled PCL powder to 98 kJ/kg while sintered HA increased the break energy to 153 kJ/kg. The blend of milled PCL with unsintered TCP also reduced the powder break energy to 92 kJ/kg while the sintered TCP increased the break energy to 136 kJ/kg. For both additives, the unsintered additive produced a powder with a reduction in break energy while the sintered additive produced a powder with an increase in break energy. As a result, the unsintered additive increases the flow of the milled material.

The unsintered HA and milled PCL blend were evaluated in a commercially available SLS printer. The printing chamber reached temperatures close to the melting temperature of the powder, and the powder still indicated good flow within the printer. The powder was used to print various test specimen.

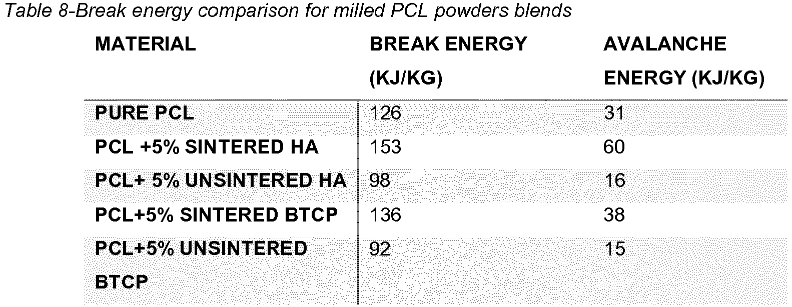

The milled PCL microparticles blended with unsintered HA were used within a commercially available SLS printer. The printing chamber reached temperatures of close to Tm, and the powder still indicated good flow within the printer. The powder was used to print various test specimen. The development cube printed with developed printing parameters can be seen in FIG 9. These powders were also quantitatively characterized for avalanche energy. The avalanche energy of the pure milled PCL powder was 31 kJ/kg. The milled PCL and unsintered HA blend saw a reduction in avalanche energy to 16 kJ/kg while the milled PCL and sintered HA blend saw an increase in avalanche energy to 60 kJ/kg. The milled PCL and unsintered TCP blend saw a reduction in avalanche energy to 15 kJ/kg while the sintered TCP blend saw an increase in avalanche energy to 38 kJ/kg. The avalanche energies of blends containing unsintered additives saw a reduction in avalanche energy while the blends containing sintered additives saw an increase in avalanche energy. As a result the unsintered additives benefitted the flow of the powder.

### EXAMPLE 4

A PLLA (RESOMER^{®}L206S) milled powder was developed by cryogenically milling a granulate form with a mill. The milling protocol utilized a milling process of which the machine rotor reached speeds of up to 12000 rpm and the door rotor reached speeds of 11000 rpm. Through this process, 90% of the irregular shaped, milled PLLA material was able to be produced below 100 µm.The d50 particle size for the milled PLLA is 54 µm. The particle size range and shape was indicated in Example 1.

Similar to Example 3 different powder mixtures were prepared using sintered HA and TCP as well as unsintered versions of HA and TCP (all additives commerically available from Himed). The bulk density increased compared to pure milled PLLA powder (0.41g/mL). The values of using unsintered TCP (0.45g/mL) and unsintered HA (0.45g/mL) were higher versus sintered TCP (0.43g/mL) and sintered HA (0.41g/mL).

The thermal characterization of the polymer blend with HA versus pure polymer showed that there is no significant difference between thermal properties of the pure polymer and the polymer blend as in the previous examples.

The pure polymer powder demonstrated a good blade test result at room temperature without the need of an additive as indicated in Example 1. Still, at elevated temperatures, the powder began to agglomerate during the printing process. Running the same experiments in a blade-based SLS led to a failed built due to bad flowability.

Flow additive was investigated in order to evaluate any benefits to powder flow. Multiple blends of PLLA milled microparticles and additives were produced. These blends are are PLLA including added amounts of unsintered HA, sintered HA, unsintered TCP, and/or sintered TCP. The material produced was intended for use within a commercially available SLS machine. The HA was added in order to improve powder flowability within the machine. Blends were mixed through tumble mixing and high shear mixing machinery. SEM imaging was used to view distribution of additive and morphology of the milled PCL powder.

SEM images indicate that the additive is adhering to the surface of the milled PLLA microparticles, and that the blend with the additive has a homogenous distribution. After the blends were prepared, a qualitative blade test was performed. From the quantitative data seen in Tables 9 and 10, HA blends did perform well compared to TCP blends. Milled PLLA blends with unsintered HA and unsintered TCP indicated the largest improvement in flow. Milled PLLA blends with sintered HA and unsintered HA were quantitatively analyzed at high temperatures. At high temperatures, the unsintered HA improves the flow of the milled PLLA powder by 45% compared to the pure material. The sintered HA also benefits the flow by a reduction in break energy of 13%.

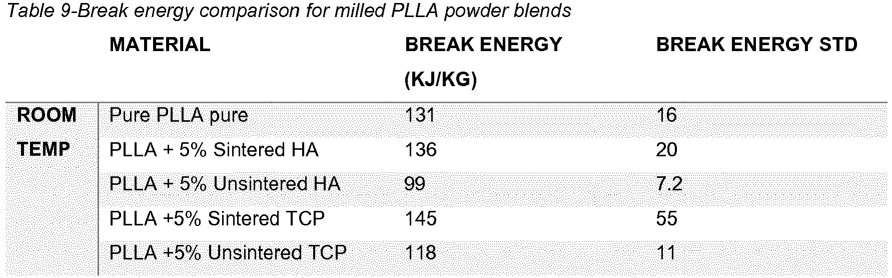

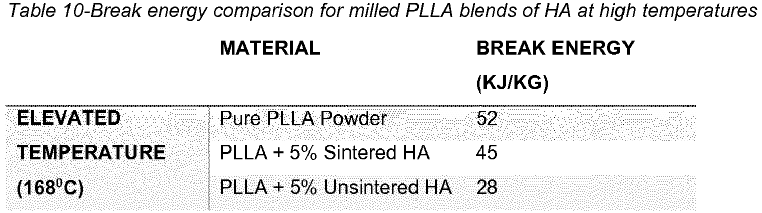

The flowablity of the powders was also checked using a roll based SLS machine. The improved flowablity of the PLLA+Unsintered HA was not sacrificed at elevated temperature within a commercially available SLS machine. Unsintered HA blends also produced multiple testing specimen.

After testing, multiple specimen were broken to view additive dispersion amongst a printed part. The unsintered HA blends showed a much more homogenous print.

### REFERENCE EXAMPLE 5

The spherical emulsion based PCL microparticles used in this example were produced using the same method as Example 2. The spherical PCL emulsion microparticles and HA additives in sintered and unsintered state were blended using tumble mixing and/or high shear mixing machinery.

Both of the emulsion based powder mixtures spread well using the qualitative blade test. Similarly the quantitative assessment of spherical PCL emulsion based powders, both the sintered and unsintered HA additives decreased the break energy of the pure material by 43-41%. The flow data of the spherical PCL emulsion particles with additive is summarized in Table 11.

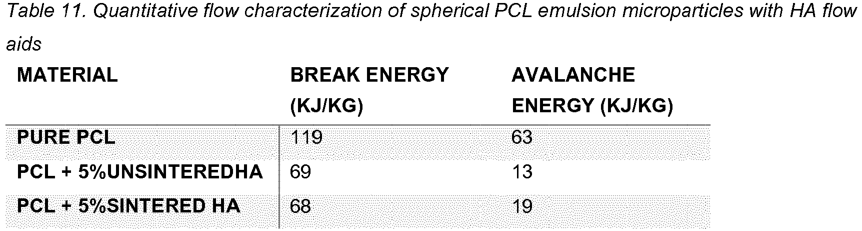

### REFERENCE EXAMPLE 6

Spherical Polylactic acid (PLLA, RESOMER^{®} L207S commercially available from Evonik) microparticles were produced using a similar solvent based emulsion process to Example 1 except including two inorganic filler materials being sintered HA (commercially available from Sigma Aldrich) and calcium carbonate (commercially available from Schaefer Kalk). 35 g of PLLA was dissolved in 301 g dichloromethane followed by addition of 7.8 g calcium carbonate and hydroxyapatite each to the same polymer solution. The mixture was slowly added to the continuous phase, which has 4.6 liter of 3 % w/w PVA in water while mixing using a homogenizer at 15000 rpm over the period of six minutes. The resulting emulsion was then passed through a packed bed column over the period of forty four minutes. The emulsion was collected in excess volume of water and allowed the particles to harden by removing the solvent. The ratio between inorganic and polymer was 30/70 wt%, which was confirmed by TGA measurements being around 27wt% inorganic.

Flowability results indicated a good flow with an angle of repose of 20.6° and a break energy of about 38kJ/kg.

### EXAMPLE 7

Polycaprolactone powder (PCL, RESOMER^{®} C212), Polylactic acid powder (PLA, RESOMER^{®} L 206 S), and Polydioxanone powder (PDO, RESOMER^{®} X 206 S) were produced using a cryogenic milling process with a mill. The particle size distributions of the powders can be seen in Table 13.

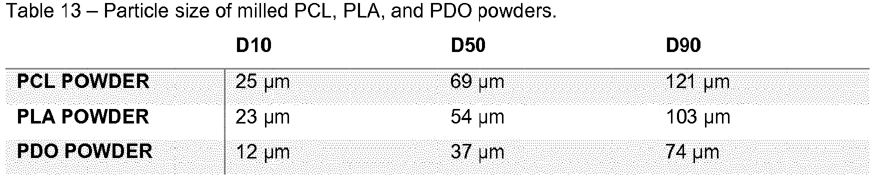

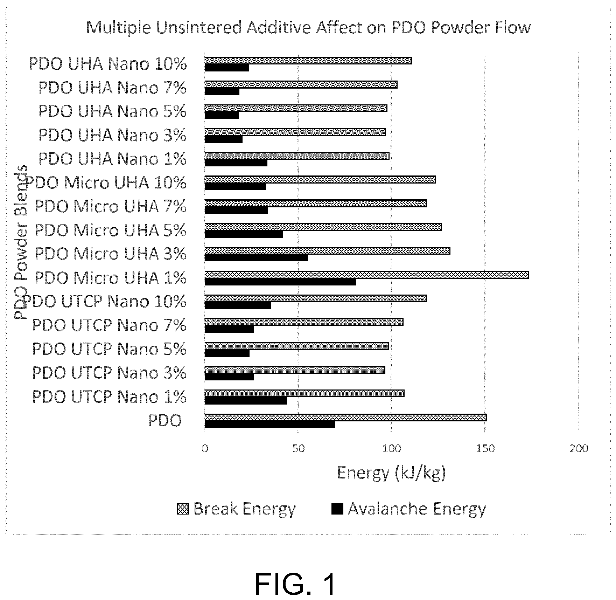

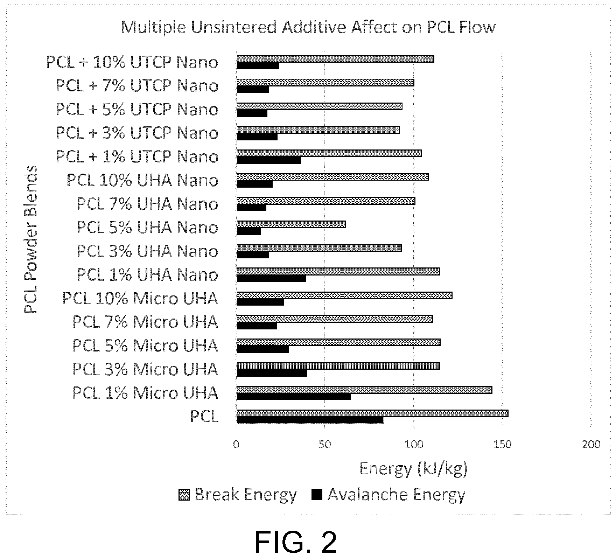

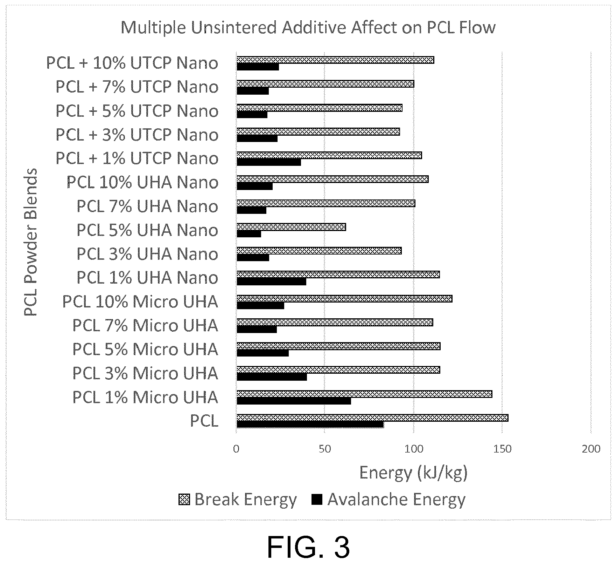

All three powders were blended with unsintered HA (UHA Nano, D50 = 0.8-0.9 µm), unsintered TCP (UTCP Nano, D 50 = 0.8-0.9 µm), and micro unsintered HA (Micro UHA, d50 = 3 µm) in weight percentages of 1, 3, 5, 7, 10 wt% additive to powder using a high shear mixer. The resulting powders were quantitatively analyzed for their flow characteristics. The graphical representation of the data can be seen in FIG. 1, FIG. 2, and FIG. 3.

All unsintered additives reduced the avalanche and break energies of the blends compared to the original polymer powder except for the 1 wt% Micro UHA blend of PDO. As a result, most of the powder blends with unsintered additives saw an improvement in flow.

### EXAMPLE 8

A Polycaprolactone powder (PCL, RESOMER^{®} C212) was created using a cryogenically mill grinding method described in Example 3. This powder was blended with unsintered HA (UHA Nano, D50 = 0.8-0.9 µm), unsintered TCP (UTCP Nano, D 50 = 0.8-0.9 µm), and micro unsintered HA (Micro UHA, d50 = 3 µm) in weight percentages of 1, 3, 5, 7, 10 wt% additive to powder using a high shear mixer. The resulting powder blends were evaluated using tap density evaluation to determine the Hausner Ratio and Compressibility Index of each powder. The Hausner Ratio is a ratio between the bulk density and the tap density of the powder. The Compressibility Index is a percentage representation of how much the powder compresses during the tap density measurement. The results can be seen in Table 14.

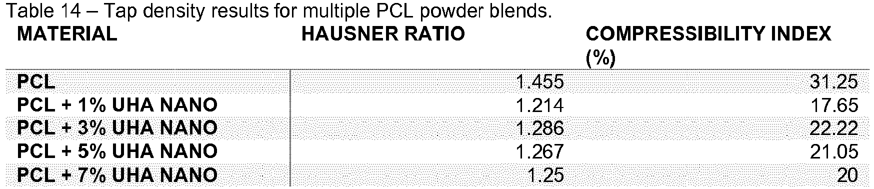

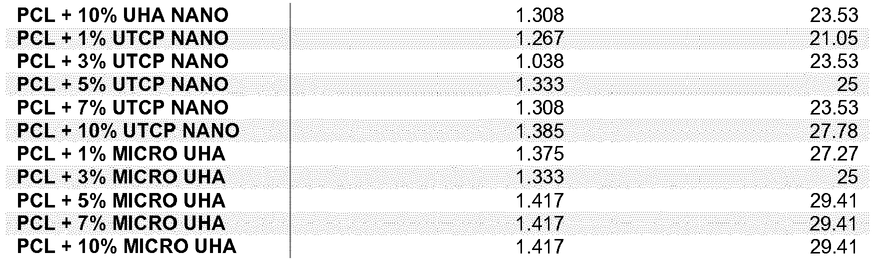

Table14 indicates that the powder blends see a reduction in Hausner Ratio and Compressibility Index for all powder blends with the unsintered additives.

### EXAMPLE 9

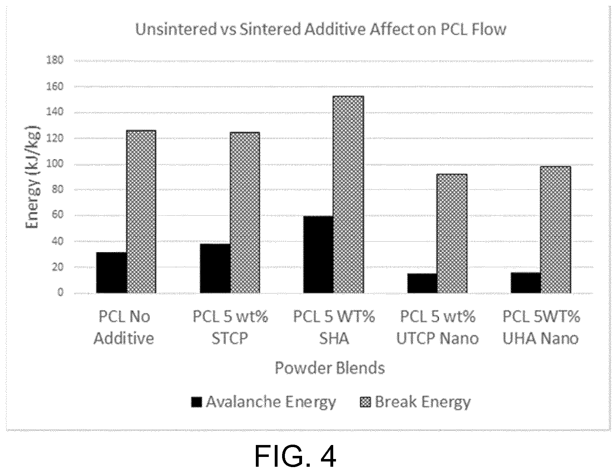

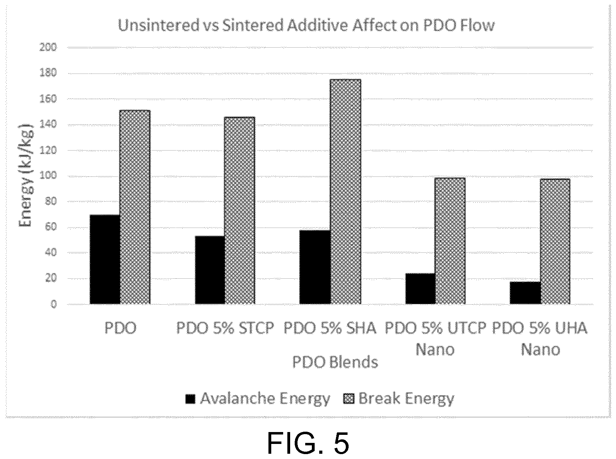

Polycaprolactone (PCL, RESOMER^{®} C209) and Polydioxanone (PDO, RESOMER^{®} X 206 S) polymers were milled using the cryogenically mill method described in Example 3. Both polymer powders were blended with 5 wt% sintered HA (SHA), sintered (STCP), unsintered TCP (UTCP Nano), and unsintered HA (UHA Nano) using a high shear mixer. All powders were quantitatively evaluated for avalanche energy and break energy using a dynamic flow analysis. The results for the dynamic flow analysis can be seen in FIG. 4 and FIG. 5.

For all powder blends, regardless of polymer composition, the unsintered additive reduced the break energy and avalanche energy of the original polymer powders. The sintered additives did not benefit the flow of the powders, as can be indicated by a non-significant change or a significant increase in the powder break energy and avalanche energy. As a result, the unsintered additives benefit the flow of the powders while the sintered additives do not.

## Claims

1. A free-flowing biocompatible polymeric powder for three-dimensional printing, comprising cryogenically ground polymeric particles having a D50 in the range of 1-150 µm and a flow aid, wherein the flow aid is a spherical free-flowing biocompatible powder, comprising polymeric particles having a D50 of 0.1 to 10 µm and whereby the D50 is determined via laser scattering.

2. A polymeric powder of milled nature, wherein the powder further comprises a flow aid, wherein the flow aid is a bioceramic, and wherein the bioceramic is in a partially amorphous biocompatible form has a D50 of 0.1 to 10 µm, whereby the D50 is determined via laser scattering.

3. Use of the polymeric powder according to claim 2 for three-dimensional printing.

4. Use according to claim 3, **characterized in that** the polymer is selected from the group consisting of polyarylketones, polymethacrylates, polycarbonates, polyacetals, polyethylenes, polypropylenes, polylactides, polydioxanones, polycaprolactones, polyesteramide, polyurethane, polytrimethyl carbonates, polyglycolide, poly(amio acid) and copolymers of the respective monomers like poly(lactide-co-glycolide), poly(lactide-co-caprolactone), poly(lactide-co-trimethly carbonate), poly(lactide-co-polyethylene-glycol), poly(orthoester), a poly(phosphazene), a poly(hydroxybutyrate) a copolymer containing a poly(hydroxybutarate), a poly(lactide-co-caprolactone), a polyanhydride, a poly(dioxanone), a poly(alkylene alkylate), a copolymer of polyethylene glycol and a polyorthoester, a biodegradable polyurethane, a polyamide, a polyetherester, a polyacetal, a polycyanoacrylate, a poly(oxyethylene)/poly(oxypropylene) copolymer, polyketals, polyphosphoesters, polyhydroxyvalerates or a copolymer containing a polyhydroxyvalerate, polyalkylene oxalates, polyalkylene succinates, poly(maleic acid), and copolymers, terpolymers, combinations thereof.

5. Use according to claim 3, **characterized in that** the polymer is selected from the group consisting of polylactides, polydioxanones, polyesteramide, polycaprolactone, polyurethane, polytrimethyl carbonate, polyglycolide, poly(amio acid) and copolymers of the respective monomers like poly(lactide-co-glycolide), poly(lactide-co-caprolactone), poly(lactide-co-trimethly carbonate), poly(lactide-co-polyethylene-glycol), poly(orthoester), a poly(phosphazene), a poly(hydroxybutyrate) a copolymer containing a poly(hydroxybutarate), a polyanhydride, a poly(alkylene alkylate), a copolymer of polyethylene glycol and a polyorthoester, a biodegradable polyurethane and it copolymers and blends thereof.

6. A composition with improved flowability for use in powder processing technologies comprising:
(a) cryogenically ground polymeric powder particle;
(b) flow aid having a D50 of 0.1 to 10 µm, wherein the flow aid is a polymeric powder that is different from the polymeric particle in (a) and
wherein the polymeric powder particle comprises a biocompatible polymer or a biodegradable polymer, whereby the D50 is determined via laser scattering.

7. A composition with improved flowability for use in powder processing technologies comprising:(a) cryogenically ground polymeric powder particle; (b) flow aid having a D50 of 0.1 to 10 µm; wherein the flow aid comprises amorphous, un-sintered calcium phosphate, and wherein the polymeric powder particle comprises a biocompatible polymer or a biodegradable polymer, whereby the D50 is determined via laser scattering.

## Patentansprüche

1. Frei fließendes biokompatibles Polymerpulver für den dreidimensionalen Druck, umfassend kryogen gemahlene Polymerteilchen mit einem D50 im Bereich von 1-150 µm und ein Fließhilfsmittel, wobei es sich bei dem Fließhilfsmittel um ein kugelförmiges freifließendes biokompatibles Pulver, das Polymerteilchen mit einem D50 von 0,1 bis 10 µm umfasst, handelt, wobei der D50 mittels Laserstreuung bestimmt wird.

2. Gemahlenes Polymerpulver, wobei das Pulver ferner ein Fließhilfsmittel umfasst, wobei es sich bei dem Fließhilfsmittel um eine Biokeramik handelt und wobei die Biokeramik in teilweise amorpher biokompatibler Form vorliegt und einen D50 von 0,1 bis 10 µm aufweist, wobei der D50 mittels Laserstreuung bestimmt wird.

3. Verwendung des Polymerpulvers nach Anspruch 2 für den dreidimensionalen Druck.

4. Verwendung nach Anspruch 3, **dadurch gekennzeichnet, dass** das Polymer aus der Gruppe bestehend aus Polyarylketonen, Polymethacrylaten, Polycarbonaten, Polyacetalen, Polyethylenen, Polypropylenen, Polylactiden, Polydioxanonen, Polycaprolactonen, Polyesteramid, Polyurethan, Polytrimethylcarbonaten, Polyglykolid, Poly(aminosäure) und Copolymeren der jeweiligen Monomere wie Poly(lactid-co-glykolid), Poly-(lactid-co-caprolacton), Poly(lactid-cotrimethylcarbonat), Poly(lactid-co-polyethylenglykol), Poly(orthoester), einem Poly(phosphazen), einem Poly(hydroxybutyrat), einem Copolymer, das ein Poly(hydroxybutyrat) enthält, einem Poly(lactid-cocaprolacton), einem Polyanhydrid, einem Poly(dioxanon), einem Poly(alkylenalkylat), einem Copolymer von Polyethylenglykol und einem Polyorthoester, einem biologisch abbaubaren Polyurethan, einem Polyamid, einem Polyetherester, einem Polyacetal, einem Polycyanoacrylat, einem Poly(oxyethylen)/Poly(oxypropylen)-Copolymer, Polyketalen, Polyphosphorestern, Polyhydroxyvaleraten oder einem Copolymer, das ein Polyhydroxyvalerat enthält, Polyalkylenoxalaten, Polyalkylensuccinaten, Poly(maleinsäure) und Copolymeren, Terpolymeren und Kombinationen davon ausgewählt ist.

5. Verwendung nach Anspruch 3, **dadurch gekennzeichnet, dass** das Polymer aus der Gruppe bestehend aus Polylactiden, Polydioxanonen, Polyesteramid, Polycaprolacton, Polyurethan, Polytrimethylcarbonat, Polyglykolid, Poly(aminosäure) und Copolymeren der jeweiligen Monomere wie Poly(lactid-co-glykolid), Poly-(lactid-co-caprolacton), Poly(lactid-cotrimethylcarbonat), Poly(lactid-co-polyethylenglykol), Poly(orthoester), einem Poly(phosphazen), einem Poly(hydroxybutyrat), einem Copolymer, das ein Poly(hydroxybutyrat) enthält, einem Polyanhydrid, einem Poly(alkylenalkylat), einem Copolymer von Polyethylenglykol und einem Polyorthoester, einem biologisch abbaubaren Polyurethan und Copolymeren und Mischungen davon ausgewählt ist.

6. Zusammensetzung mit verbesserter Fließfähigkeit zur Verwendung bei Pulververarbeitungstechnologien, umfassend:
(a) kryogen gemahlenes Polymerpulverteilchen;
(b) Fließhilfsmittel mit einem D50 von 0,1 bis 10 µm, wobei es sich bei dem Fließhilfsmittel um ein von dem Polymerpartikel in (a) verschiedenes Polymerpulver handelt und wobei das Polymerpulverteilchen ein biokompatibles Polymer oder ein biologisch abbaubares Polymer umfasst, wobei der D50 mittels Laserstreuung bestimmt wird.

7. Zusammensetzung mit verbesserter Fließfähigkeit zur Verwendung bei Pulververarbeitungstechnologien, umfassend: (a) kryogen gemahlenes Polymerpulverteilchen; (b) Fließhilfsmittel mit einem D50 von 0,1 bis 10 µm; wobei das Fließhilfsmittel amorphes, ungesintertes Calciumphosphat umfasst und wobei das Polymerpulverteilchen ein biokompatibles Polymer oder ein biologisch abbaubares Polymer umfasst, wobei der D50 mittels Laserstreuung bestimmt wird.

## Revendications

1. Poudre polymérique biocompatible à écoulement libre pour l'impression tridimensionnelle, comprenant des particules polymériques broyées de manière cryogénique possédant un D50 dans la plage de 1 à 150 µm et un auxiliaire d'écoulement, l'auxiliaire d'écoulement étant une poudre biocompatible à écoulement libre sphérique, comprenant des particules polymériques possédant un D50 de 0,1 à 10 µm et le D50 étant déterminé via diffusion laser.

2. Poudre polymérique de nature broyée, la poudre comprenant en outre un auxiliaire d'écoulement, l'auxiliaire d'écoulement étant une biocéramique, et la biocéramique étant sous une forme biocompatible partiellement amorphe possédant un D50 de 0,1 à 10 µm, le D50 étant déterminé via diffusion laser.

3. Utilisation de la poudre polymérique selon la revendication 2 pour l'impression tridimensionnelle.

4. Utilisation selon la revendication 3, **caractérisée en ce que** le polymère est choisi dans le groupe constitué par des polyarylcétones, des polyméthacrylates, des polycarbonates, des polyacétals, des polyéthylènes, des polypropylènes, des polylactides, des polydioxannones, des polycaprolactones, un polyesteramide, un polyuréthane, des polytriméthyl-carbonates, un polyglycolide, un poly(acide aminé) et des copolymères des monomères respectifs comme un poly(lactide-coglycolide), un poly(lactide-co-caprolactone), un poly(lactide-co-triméthyl-carbonate), un poly(lactide-co-polyéthylène-glycol), un poly(orthoester), un poly(phosphazène), un poly(hydroxybutyrate), un copolymère contenant un poly(hydroxybutarate), un poly(lactide-co-caprolactone), un polyanhydride, une poly(dioxanone), un poly(alkylate d'alkylène), un copolymère de polyéthylène glycol et d'un polyorthoester, un polyuréthane biodégradable, un polyamide, un polyétherester, un polyacétal, un polycyanoacrylate, un copolymère de poly(oxyéthylène)/poly(oxypropylène), des polycétals, des polyphosphoesters, des polyhydroxyvalérates ou un copolymère contenant un polyhydroxyvalérate, des poly(oxalate d'alkylène), des poly(succinate d'alkylène), poly(acide maléique), et des copolymères, des terpolymères et des combinaisons correspondantes.

5. Utilisation selon la revendication 3, **caractérisée en ce que** le polymère est choisi dans le groupe constitué par des polylactides, des polydioxanones, un polyesteramide, une polycaprolactone, un polyuréthane, un polytriméthyl-carbonate, un polyglycolide, un poly(acide aminé) et des copolymères des monomères respectifs comme un poly(lactide-co-glycolide), un poly(lactide-co-caprolactone), un poly(lactide-cotriméthyl-carbonate), un poly(lactide-co-polyéthylèneglycol), un poly(orthoester), un poly(phosphazène), un poly(hydroxybutyrate), un copolymère contenant un poly(hydroxybutarate), un polyanhydride, un poly(alkylate d'alkylène), un copolymère de polyéthylène glycol et d'un polyorthoester, un polyuréthane biodégradable, et ses copolymères et mélanges correspondants.

6. Composition dotée d'une aptitude à l'écoulement améliorée pour une utilisation dans des technologies de traitement de poudre comprenant :
(a) une particule de poudre polymérique broyée de manière cryogénique ;
(b) un auxiliaire d'écoulement possédant un D50 de 0,1 à 10 µm, l'auxiliaire d'écoulement étant une poudre polymérique qui est différente de la particule polymérique en (a) et
la particule de poudre polymérique comprenant un polymère biocompatible ou un polymère biodégradable, le D50 étant déterminé via diffusion laser.

7. Composition dotée d'une aptitude à l'écoulement améliorée pour une utilisation dans des technologies de traitement de poudre comprenant : (a) une particule de poudre polymérique broyée de manière cryogénique ; (b) un auxiliaire d'écoulement possédant un D50 de 0,1 à 10 µm ; l'auxiliaire d'écoulement comprenant du phosphate de calcium amorphe, non fritté, et la particule de poudre polymérique comprenant un polymère biocompatible ou un polymère biodégradable, le D50 étant déterminé via diffusion laser.
